# EUROPEAN PATENT APPLICATION

(11) **EP 3 533 790 A2**
(43) Date of publication of application: **04.09.2019**
(21) Application number: 19160444.6
(22) Date of filing: 04.03.2019
(51) Int. Cl.: C07D 279/28

(54) **AN IMPROVED PROCESS FOR PREPARATION OF CHLORPROMAZINE OR ITS PHARMACEUTICALLY ACCEPTABLE SALTS**

(30) Priority: 03.03.2018 IN 201841007953
(71) Applicant: Solara Active Pharma Sciences Limited, Chennai 600127 (IN)
(72) Inventor: Sankar, Arjunan, 600048 Chennai (IN); Senthilnathan, Palanivel, 600073 Chennai (IN); Aramanai Laksmanan, Srinivasan, 612001 Kumbakonam (IN); Adhimoolam, Thirumalai, 605754 Villupuram (District) (IN); Prasad Krishnan, Devendra, 635123 Dharmapuri (IN); Kumar Ray, Uttam, 600127 Chennai (IN); Vittal, Tangirala, 600100 Chennai (IN)
(74) Representative: AWA Denmark A/S

(57) **Abstract**

The present invention relates to an improved process for the preparation of Chlorpromazine or its pharmaceutically acceptable salts thereof, preferably process for the preparation of Chlorpromazine or its pharmaceutically acceptable salts thereof having high purity.

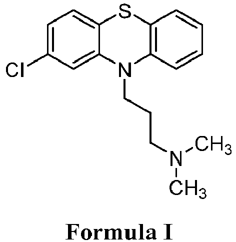

## Description

### RELATED PATENT APPLICATION

This application claims the priority to and benefit of Indian Patent Application No. 201841007953 filed on March 03, 2018; the disclosures of which are incorporated herein by reference.

### FIELD OF THE INVENTION

The present invention relates to an improved process for the preparation of Chlorpromazine or its pharmaceutically acceptable salts thereof, preferably process for the preparation of Chlorpromazine or its pharmaceutically acceptable salts thereof having high purity.

### BACKGROUND OF THE INVENTION

Chlorpromazine and its pharmaceutically acceptable salts are approved as an antipsychotic drugs, marketed in United States under the trade name Thorazine. Chlorpromazine is chemically known as 2-chloro-10-[3-(dimethylamino) propyl]-phenothiazine, having the formula I as mentioned below.

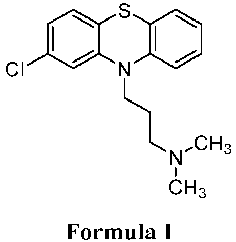

Chlorpromazine was first disclosed in the US Patent No. 2,645,640. This patent discloses the process for preparation of Chlorpromazine involving the alkylation reaction of 2-chlorophenothiazine with 3-dimethylaminopropylchloride in the presence of base such as sodamide in non-aqueous solvents such as xylene or toluene as shown below.

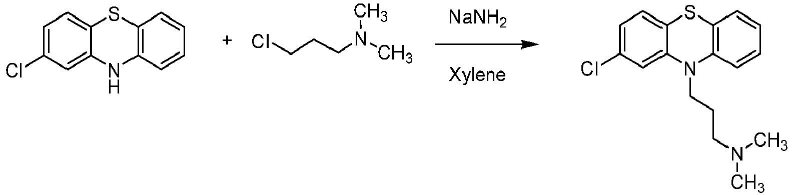

The publication S J Scholka & H Zimmer, Synthesis 1984, page-29 discloses the process for preparation of Chlorpromazine involving the alkylation reaction of 2-chlorophenothiazine with 3-dimethylaminopropylchloride in presence of base such as anhydrous potassium carbonate and sodium hydroxide and a phase transfer catalyst such as tetra-n-butyl ammonium hydrogen sulfate in non-aqueous solvents such as toluene as shown below.

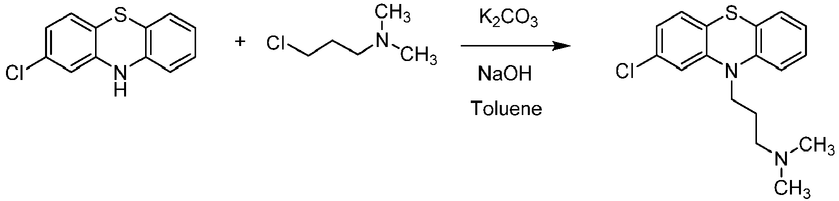

The publication Chem. Pharm. Bull. 33(11)5108-5109 (1985), discloses the process for preparation of Chlorpromazine involving the alkylation reaction of 2-chlorophenothiazine with 3-dimethylaminopropylchloride hydrochloride in the presence of potassium hydroxide and aliquat without organic solvents as shown below.

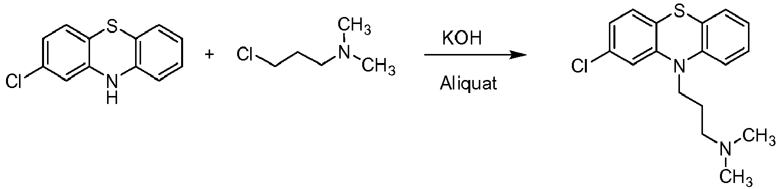

The Chinese Patent CN 102617509B discloses the process for preparation of Chlorpromazine involving the alkylation reaction of 2-chlorophenothiazine with 3-dimethyl aminopropylchloride in presence of sodium hydroxide and tert-butylammonium bromide in toluene as shown below.

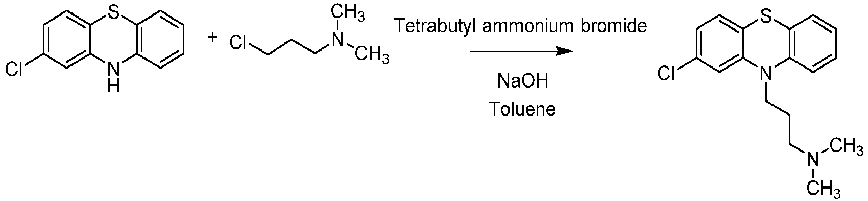

The procedure herein involves the heating of 2-chlorophenothiazine with toluene and water followed by drying the same under reduced pressure. The dried reaction mixture was refluxed with sodium hydroxide, tert-butylammonium bromide and toluene for dehydration then toluene solution of 3-dimethylaminopropylchloride was added to the reaction mixture to obtain the Chlorpromazine. The process involves the removal of water before alkylation reaction of 2-chlorophenothiazine with 3-dimethylaminopropylchloride.

The publication Tetrahedron Letters No.10, 763-766, 1969; discloses the formation of dimer by the oxidation of 2-chlorophenothiozine in dimethyl sulfoxide as shown below.

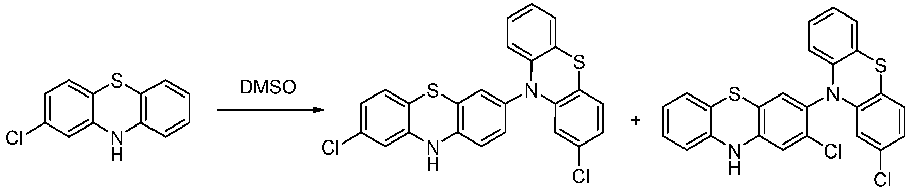

The publication Journal of Pharmaceutical Sciences Vol. 66, No. 10, October 1922, 1395-1398; discloses the formation of dimer by the oxidation of 2-chlorophenothiozine in acetic medium.

Besides the existence of various processes for the preparation of Chlorpromazine or its pharmaceutically acceptable salts there remains a need for improved processes for the preparation of Chlorpromazine or its pharmaceutically acceptable salts.

### OBJECTIVE OF THE INVENTION

The objective of the present invention is to provide an improved process for the preparation of Chlorpromazine or its pharmaceutically acceptable salts having high purity.

The another objective of the present invention is to provide an improved process for the preparation of Chlorpromazine or its pharmaceutically acceptable salts having the dimeric impurities less than 0.05% w/w.

The present inventors of the invention provides an improved process for the preparation of Chlorpromazine or its pharmaceutically acceptable salts having high purity and that process is economically significant and industrially viable.

### SUMMARY OF THE INVENTION

Accordingly, there is provided an improved process for the preparation of Chlorpromazine or its pharmaceutically acceptable salts.

One aspect of the present invention is to provide an improved process for the preparation of Chlorpromazine of formula I or its pharmaceutically acceptable salts, 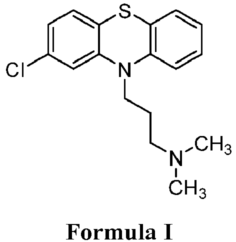 said process comprising reacting 2-chlorophenothiazine compound of formula II with a compound of formula III wherein X is selected from H, Cl, Br or I 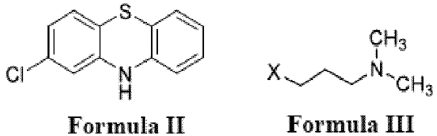 in the presence of base in a biphasic medium to obtain the compound of formula I or its pharmaceutically acceptable salts.

In some embodiment of the present invention, in the above described process for preparing Chlorpromazine or its pharmaceutically acceptable salts, the said base is selected from the group comprising of sodium hydroxide, potassium hydroxide, lithium hydroxide, magnesium hydroxide; carbonate or bicarbonates of sodium, potassium, magnesium, lithium and barium or aqueous mixture thereof; preferably aqueous potassium hydroxide.

In some embodiment of the present invention, in the above described process for preparing Chlorpromazine or its pharmaceutically acceptable salts, the said biphasic medium is selected from the group comprising of aqueous phase, non-aqueous phase selected from dichloromethane, dichloroethane, chloroform, carbon tetrachloride, tetrahydrofuran, diethyl ether, diisopropyl ether, dimethyl ether, benzene, toluene, cyclohexane, xylene or mixture thereof.

Another aspect of the present invention is to provide a process for the purification of Chlorpromazine hydrochloride of formula Ia 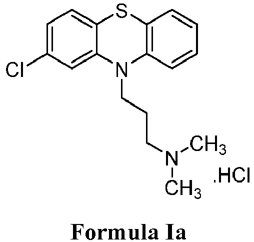 said process comprising the steps of:
(i) providing a solution of chlorpromazine hydrochloride of formula Ia in one or more organic solvent;
(ii) optionally concentrating the solution obtained in step (i) under reduced pressure;
(iii) adding ester solvent to the solution or suspension obtained in step (i) or (ii);
(iv) optionally cooling the step (iii); and
(v) isolating the Chlorpromazine hydrochloride of formula Ia from the solution or suspension obtained in step (iii) or (iv).

In some embodiment of the present invention, in the above described process for purification of Chlorpromazine hydrochloride, the said organic solvent selected from the group comprising of xylene, toluene, benzene, hexane, cyclohexane, methyl cyclohexane, dichloromethane, chloroform, carbon tetrachloride, dichloromethane, dichloroethane, dimethyl formamide, acetone, methanol, ethanol, isopropanol, sulfolane, dimethyl sulfoxide or mixture thereof; preferably mixture of toluene and methanol.

In some embodiment of the present invention, in the above described process for purification of Chlorpromazine hydrochloride, the said ester solvent selected from the group comprising of ethyl acetate, butyl acetate, methyl acetate, isopropyl acetate, isoamyl acetate, ethyl butyrate, ethyl acetoacetate or mixture thereof; preferably ethyl acetate.

Another aspect of the present invention is to provide a process for the preparation of Chlorpromazine hydrochloride compound of formula Ia having less than 0.05% w/w of dimeric impurities of formula IVa or IVb 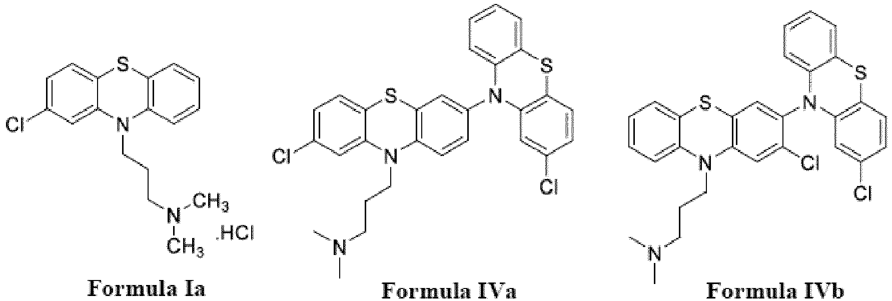 said process comprising the steps of:
i. reacting 2-chlorophenothiazine compound of formula II with 3-dimethylaminopropylchloride compound of formula IIIa 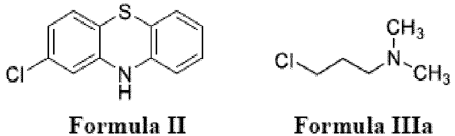 in the presence of base in a biphasic medium to obtain Chlorpromazine;
ii. providing a solution of Chlorpromazine in one or more organic solvent;
iii. adding alcoholic hydrochloric acid to the solution obtained in step (ii);
iv. isolating the Chlorpromazine hydrochloride of formula Ia from the solution obtained in step (iii); and
v. optionally purifying the Chlorpromazine hydrochloride with one or more organic solvent.

In some embodiment of the present invention, there is provided a process for the preparation of Chlorpromazine hydrochloride having the dimeric impurities of formula IVa or IVb less than 0.02% w/w.

In some embodiment of the present invention, in the above described process for the preparation of Chlorpromazine hydrochloride, the said base selected from the group comprising of sodium hydroxide, potassium hydroxide, lithium hydroxide, magnesium hydroxide; carbonate or bicarbonates of sodium, potassium, magnesium and lithium or aqueous mixture thereof; preferably aqueous potassium hydroxide.

In some embodiment of the present invention, in the above described process for the preparation of Chlorpromazine hydrochloride, the said biphasic medium is selected from the group comprising of aqueous phase, non-aqueous phase selected from dichloromethane, dichloroethane, chloroform, carbon tetrachloride, tetrahydrofuran, diethyl ether, diisopropyl ether, dimethyl ether, benzene, toluene, cyclohexane, xylene or mixture thereof.

In some embodiment of the present invention, in the above described process for the preparation of Chlorpromazine hydrochloride, the said organic solvent in step (ii) is selected from the group comprising of xylene, toluene, benzene, hexane, cyclohexane, methyl cyclohexane, dichloromethane, chloroform, carbon tetrachloride, dichloromethane, dichloroethane, dimethyl formamide, acetone, methanol, ethanol, isopropanol, sulfolane, dimethyl sulfoxide or mixture thereof; preferably mixture of toluene and methanol.

In some embodiment of the present invention, in the above described process for the preparation of Chlorpromazine hydrochloride, the said alcoholic hydrochloric acid is selected from the group comprising hydrochloric acid of methanol, ethanol or isopropanol; preferably methanolic hydrochloric acid.

Another aspect of the present invention is to provide an improved process for the preparation of compound of formula Ic wherein R₁ and R₂ is selected from H and CH₃; R₃ is selected from H, Cl, Br, CF₃, CH₃ and OCH₃; R₄ is selected from substituted or unsubstituted of C₁-C₄ as alkyl comprising methylene, ethylene, propylene or butylene; X is selected from H, Cl, Br or I; 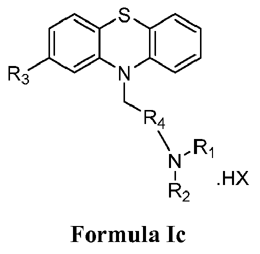 said process comprising the steps of:
a) reacting the compound of formula IIa wherein R₃ is selected from H, Cl, Br, CF₃, CH₃ and OCH₃ 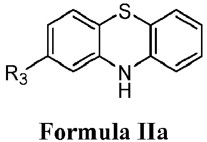 with the compound of formula IIIa wherein R₁ and R₂ is selected from H and CH₃; R₄ is selected from substituted or unsubstituted C₁-C₄ alkyl comprising methylene, ethylene, propylene or butylene; X is selected from H, Cl, Br, and I 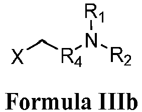 in the presence of base in a biphasic medium to obtain a compound of formula Ib; and 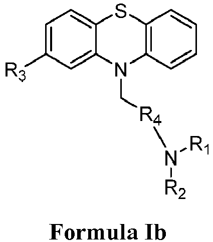
b) converting the compound of formula 1b to the compound of formula 1c.

In some embodiment of the present invention, the above described process includes compound of formula Ic selected from Promethazine, Alimemazine, Acepromazine, Promazine, Chlorpromazine or the like .

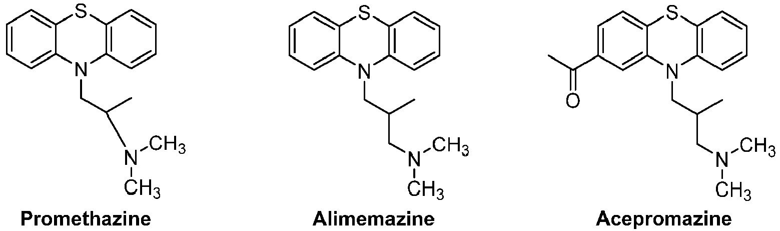

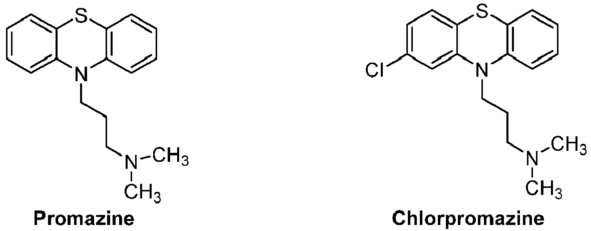

In some embodiment of the present invention, in the above described process for preparing the compound of formula Ic, the said base is selected from the group comprising of sodium hydroxide, potassium hydroxide, lithium hydroxide, magnesium hydroxide; carbonate or bicarbonates of sodium, potassium, magnesium and lithium or its aqueous mixture thereof; preferably aqueous potassium hydroxide.

In some embodiment of the present invention, in the above described process for preparing the compound of formula Ic the said biphasic medium is selected from the group comprising of aqueous phase, non-aqueous phase selected from dichloromethane, dichloroethane, chloroform, carbon tetrachloride, tetrahydrofuran, diethyl ether, diisopropyl ether, dimethyl ether, benzene, toluene, cyclohexane, xylene or mixture thereof.

### BRIEF DESCRIPTION OF THE DRAWING

Fig-1 illustrates the crystalline PXRD of Chlorpromazine hydrochloride obtained in Example-2.

### DETAILED DESCRIPTION OF THE INVENTION

The present invention provides an improved process for the preparation of Chlorpromazine or its pharmaceutically acceptable salts.

One aspect of the present invention provides an improved process for the preparation of Chlorpromazine of formula I or its pharmaceutically acceptable salts 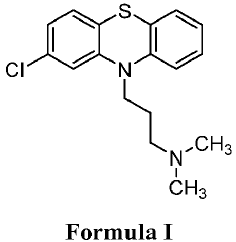 said process comprising reacting 2-chlorophenothiazine compound of formula II with a compound of formula III wherein X is selected from H, Cl, Br or I 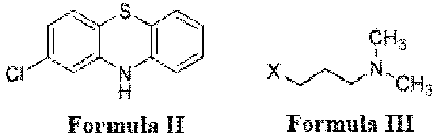 in the presence of base in a biphasic medium to obtain the compound of formula I or its pharmaceutically acceptable salts.

In some embodiment of the present invention, the base employed in the above described process for the preparation of chlorpromazine or it's pharmaceutically acceptable salts is selected from the group including but not limited to sodium hydroxide, potassium hydroxide, lithium hydroxide, magnesium hydroxide; carbonate or bicarbonates of sodium, potassium, magnesium and lithium or its aqueous mixtures thereof; preferably aqueous potassium hydroxide.

Accordingly, the present invention involves reaction of compound of formula II with the compound of formula III in presence of base in biphasic solvent system, of which one of the phase is aqueous phase. The non-aqueous phase solvent comprises of chlorinated solvents, ethers, aromatic hydrocarbons or mixtures thereof. Examples of such solvents include but not limited to dichloromethane, dichloroethane, chloroform, carbon tetrachloride, tetrahydrofuran, diethyl ether, diisopropyl ether, dimethyl ether, benzene, toluene, cyclohexane xylene or mixture thereof. Preferably aromatic hydrocarbons and more preferably toluene.

Another aspect of the present invention provides the process for the purification of chlorpromazine hydrochloride of formula Ia 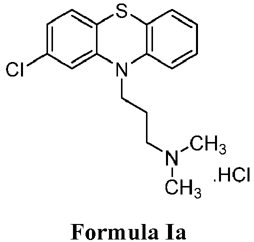 comprising the steps of:
(i) providing a solution of chlorpromazine hydrochloride of formula Ia in one or more organic solvent;
(ii) optionally concentrating the solution obtained in step (i) under reduced pressure;
(iii) adding ester solvent to the solution or suspension obtained in step (i) or (ii);
(iv) optionally cooling the step (iii); and
(v) isolating the Chlorpromazine hydrochloride of formula Ia from the solution or suspension obtained in step (iii) or (iv).

In some embodiment of the present invention, the organic solvent employed in step (i) of the above described process for the purification of chlorpromazine hydrochloride may include but not limited to xylene, toluene, benzene, hexane, cyclohexane, methyl cyclohexane, dichloromethane, chloroform, carbon tetrachloride, dichloromethane, dichloroethane, dimethyl formamide, acetone, methanol, ethanol, isopropanol, sulfolane, dimethyl sulfoxide or mixture thereof, preferably mixture of toluene and methanol.

In some embodiment of the present invention, the ester solvent employed in step (iii) of the above described process for the purification of chlorpromazine hydrochloride may include but not limited to ethyl acetate, butyl acetate, methyl acetate, isopropyl acetate, isoamyl acetate, ethyl butyrate, ethyl acetoacetate or mixture thereof; preferably ethyl acetate.

In some embodiment of the present invention, the isolation employed in step (v) is carried out by technique or methods including but not limited to cooling, filtering, washing, drying or the combination thereof.

Another aspect of the present invention provides the process for the preparation of Chlorpromazine hydrochloride compound of formula Ia having less than 0.05% w/w of dimeric impurities of formula IVa or IVb 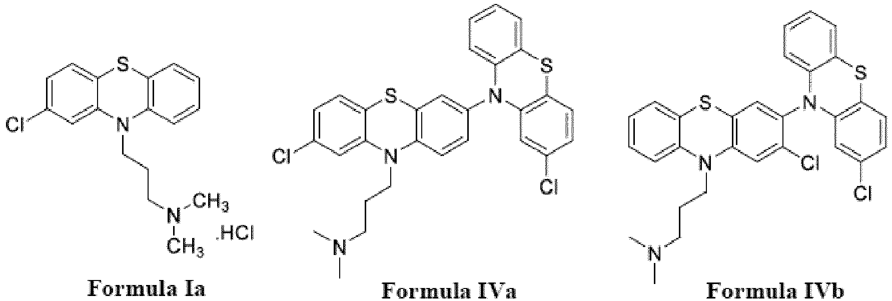 said process comprising the steps of:
i. reacting 2-chlorophenothiazine compound of formula II with 3-dimethylaminopropylchloride compound of formula IIIa 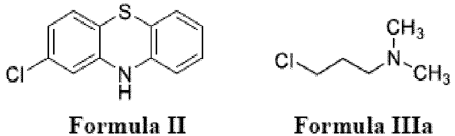 in the presence of base in biphasic medium to obtain Chlorpromazine;
ii. providing a solution of Chlorpromazine in one or more organic solvent;
iii. adding alcoholic hydrochloric acid to the solution obtained in step (ii);
iv. isolating the Chlorpromazine hydrochloride of formula Ia from the solution obtained in step (iii); and
v. optionally purifying the chlorpromazine hydrochloride with with one or more organic solvent.

In some embodiment, there is provided a process for the preparation of Chlorpromazine hydrochloride having the dimeric impurities of formula IVa or IVb less than 0.02% w/w.

In some embodiment of the present invention, the base may employed in the above described process for preparation of chlorpromazine hydrochloride may include but not limited to sodium hydroxide, potassium hydroxide, lithium hydroxide, magnesium hydroxide; carbonate or bicarbonates of sodium, potassium, magnesium and lithium or aqueous mixture thereof; preferably aqueous potassium hydroxide.

The inventors of the present invention observed that, the formation of chlorophenothiazine dimer of formula IVa and IVb during the preparation of Chlorpromazine or its pharmaceutically acceptable salts. Removal of dimeric impurity is cumbersome after the preparation of Chlorpromazine from the reaction of chlorophenothiazine with 3-dimethylaminopropylchloride in the presence of base in organic solvent such as toluene or xylene.

Surprisingly, the inventors of the present invention found that the reaction of 2-chlororphenothiazine compound of formula II with 3-dimethylaminopropylchlorid compound of formula IIIa in the presence of base in biphasic medium reduces the formation of dimeric impurity. Further, the use of biphasic medium also decreases the reaction time.

Accordingly, step (i) of the present invention involves reaction of compound of formula II with the compound of formula IIIa in presence of base in biphasic solvent system, of which one of the phase is aqueous phase. The non-aqueous phase solvent comprises of chlorinated solvents, ethers, esters, aromatic hydrocarbons or mixture thereof. Examples of such solvents include but not limited to dichloromethane, dichloroethane, chloroform, carbon tetrachloride, tetrahydrofuran, diethyl ether, diisopropyl ether, dimethyl ether, ethyl acetate, butyl acetate, methyl acetate, benzene, toluene, cyclohexane, xylene or mixture thereof. Preferably aromatic hydrocarbons and more preferably toluene.

In some embodiment of the present invention, the organic solvents employed in step (ii) of the above described process for preparing chlorpromazine hydrochloride may include but not limited to toluene, xylene, benzene or mixture thereof and preferably toluene.

In some embodiment of the present invention, the alcoholic hydrochloric acid employed in step (iii) of the above described process for preparing chlorpromazine hydrochloride may include but not limited to methanol, ethanol or isopropanol preferably methanolic hydrochloric acid. In some embodiment of the present invention, the isolation employed in step (iv) is carried out by technique or methods including but not limited to cooling, filtering, washing, drying or the combination thereof.

In some embodiment of the present invention, the organic solvent employed in step (v) of the above described process for preparing chlorpromazine hydrochloride may include but not limited to xylene, toluene, benzene, hexane, cyclohexane, methyl cyclohexane, dichloromethane, chloroform, carbon tetrachloride, dichloromethane, dichloroethane, dimethyl formamide, acetone, methanol, ethanol, isopropanol, sulfolane, dimethyl sulfoxide or mixture thereof, preferably mixture of toluene and methanol.

In some embodiment of the invention, the Chlorpromazine hydrochloride obtained is crystalline characterized by PXRD as illustrated in Figure-1.

Another aspect of the present invention provides an improved process for the preparation of compound of formula Ic wherein R₁ and R₂ is selected from H or CH₃; R₃ is selected from H, Cl, Br, CF₃, CH₃ or OCH₃; R₄ is selected from substituted or unsubstituted of C₁-C₄ alkyl such as methylene, ethylene, propylene or butylene; X is selected from H, Cl, Br or I 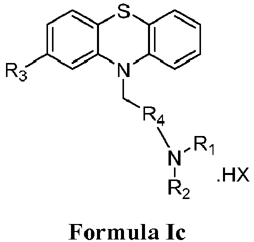 said process comprising the step of:
a) reacting a compound of formula IIa wherein R₃ is selected from H, Cl, Br, CF₃, CH₃ or OCH₃ 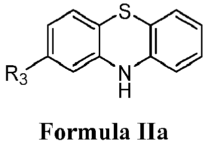 with a compound of formula IIIb wherein R₁ and R₂ is selected from H or CH₃; R₄ is selected from substituted or unsubstituted C₁-C₄ such as methylene, ethylene, propylene or butylene; X is selected from H, Cl, Br or I 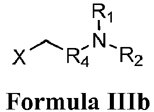 in the presence of base in a biphasic medium to obtain a compound of formula Ib; 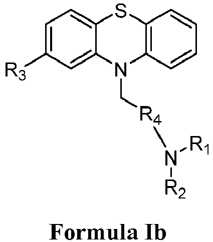
b) converting the compound of formula 1b to the compound of formula 1c.

In some embodiment of the present invention, in the above described process the compound of formula Ic is selected from Promethazine, Alimemazine, Acepromazine, Promazine, Chlorpromazine or the like.

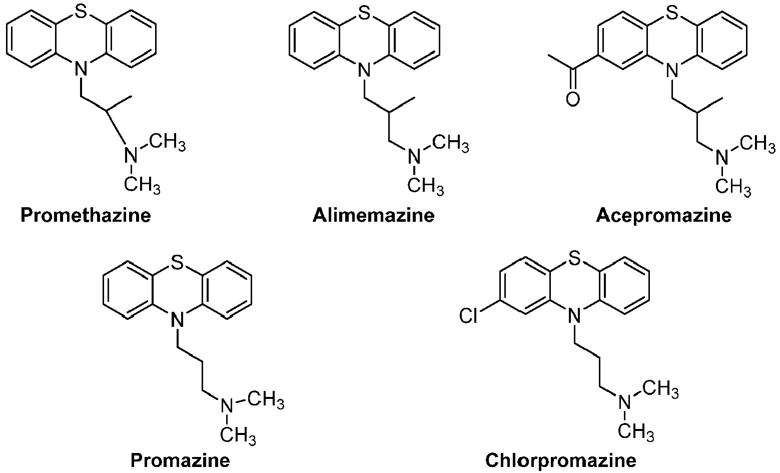

In some embodiment of the present invention, the base employed in the above described process for preparing the compound of formula Ic may include but not limited to hydroxides of sodium hydroxide, potassium hydroxide, lithium hydroxide, magnesium hydroxide; carbonate or bicarbonates of sodium, potassium, magnesium and lithium or aqueous mixture thereof; preferably aqueous potassium hydroxide.

Accordingly, the present invention involves reaction of compound of formula IIa with the compound of formula IIIb in presence of base in biphasic solvent system, of which one of the phase is aqueous phase. The non-aqueous phase solvent comprises of chlorinated solvents, ethers, esters, aromatic hydrocarbons or mixtures thereof. Examples of such solvents include but not limited to dichloromethane, dichloroethane, chloroform, carbon tetrachloride, tetrahydrofuran, diethyl ether, diisopropyl ether, dimethyl ether, ethyl acetate, butyl acetate, methyl acetate, benzene, toluene, cyclohexane xylene or mixture thereof. Preferably aromatic hydrocarbons and more preferably toluene.

The present invention is explained in detail with reference to the following examples described below, which are given for the purpose of illustration only and are not intended to limit the scope of the invention.

### EXAMPLE:

### Example 1: Preparation of Chlorpromazine Hydrochloride of formula I:

To a mixture of 2-chlorophenothiazine compound of formula II (100 g) and toluene (450 ml), aqueous potassium hydroxide (96.02 g of potassium hydroxide in 100 ml of water) was added at 30°C and then heated to 98°C. A toluene solution of 3-dimethylaminopropylchloride of formula III (135.24 g of 3-dimethylaminopropylchloride in 200 ml of toluene) was added to the heated reaction mixture at 98°C and maintained for 6 hours. The progress of the reaction was monitored by TLC. After the completion of the reaction, the reaction mass was cooled to 40°C and quenched with water (1000 ml). The organic layer was separated, from the reaction mass and washed with water (1000 ml) then concentrated to obtain a residue. The residue was dissolved in toluene (400 ml) and the contents were extracted with an aqueous solution of 0.5N hydrochloric acid (1000 ml). Toluene (100 ml) was added to the obtained aqueous hydrochloride solution containing Chlorpromazine, followed by the addition 30% sodium hydroxide solution (90 ml). The organic layer was then separated and washed with water (300 ml). Activated charcoal (Pencarb-A, 10 gm) was added to the washed organic layer and stirred for 30 minutes at 30°C. The contents were then filtered and the filtrate was concentrated under reduced pressure to obtain a residue. Toluene (700 ml) was added to the residue at 65°C and then cooled to 30°C. Methanolic hydrochloride solution (71.15 g (29.4% w/v)) was slowly added to the cooled mixture, stirred for 15 minutes at 30°C and the mass was concentrated under vacuum at 70°C. Ethyl acetate (600 ml) was added to the concentrated residue at 60°C and stirred for 1 hour at 75°C. The contents were cooled to 30°C and stirred for 90 minutes at the same temperature. The resulting solid was filtered and washed with ethyl acetate (200 ml). The wet solid was dissolved in a mixture of toluene (600 ml) and methanol (100 ml) and concentrated under vacuum. Ethyl acetate (500 ml) was added to the concentrate at 60°C and stirred for one hour at 75°C. The contents were cooled to 30°C and stirred for 2 hours at the same temperature. The resulted solid was filtered, washed with ethyl acetate (100 ml) and dried to obtain the desired Chlorpromazine hydrochloride. Yield: 82.24 %; Dimeric impurity: 0.05% w/w.

### Example 2: Preparation of Chlorpromazine Hydrochloride of formula I:

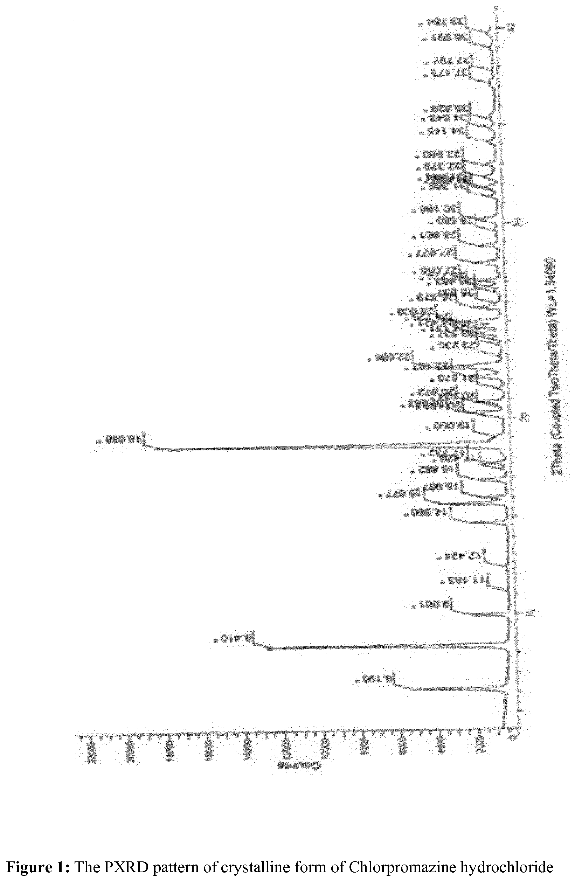

To a mixture of reaction 2-chlorophenothiazine compound of formula II (150 kg) and toluene (675 Lts), aqueous potassium hydroxide (144 Kg in 150 Lts of water) was added at 30°C and then heated to 98° C. Toluene solution of 3-dimethylaminopropylchloride (202.5 kg) was added to the reaction mixture and the progress of the reaction was monitored by HPLC. After completion of the reaction, reaction mass was treated with acid-base workup followed by activated carbon treatment. Methanolic hydrochloride solution (133. Kg) was added to resulted reaction mass followed by concentration to obtain crude Chlorpromazine hydrochloride (200 Kg). The crude Chlorpromazine hydrochloride was crystallized in a mixture of methanol (900 Lts) and toluene (150 Lts) at 60-75°C then filtered, washed with ethyl acetate and dried. The obtained crystalline form of Chlorpromazine hydrochloride was characterized by PXRD and the same has been illustrated as Figure 1. Yield: 137.4 kg; Purity: 99.8%; Dimeric impurity: 0.02% w/w.

## Claims

1. A process for the preparation of Chlorpromazine of formula I or its pharmaceutically acceptable salts 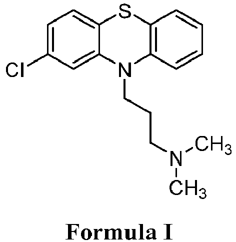 said process comprising reacting 2-chlorophenothiazine compound of formula II with a compound of formula III wherein X is selected from H, Cl, Br or I 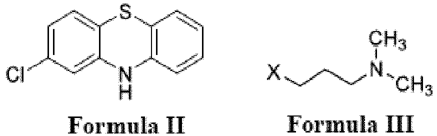 in the presence of base in a biphasic medium to obtain the compound of formula I.

2. The process as claimed in claim 1, wherein said base is selected from the group comprising of sodium hydroxide, potassium hydroxide, lithium hydroxide, magnesium hydroxide, barium hydroxide; carbonate or bicarbonates of sodium, potassium, magnesium, lithium and barium or aqueous mixture thereof; and preferably aqueous potassium hydroxide.

3. The process as claimed in claim 1, wherein said biphasic medium is selected from the group comprising of aqueous phase, non-aqueous phase selected from dichloromethane, dichloroethane, chloroform, carbon tetrachloride, tetrahydrofuran, diethyl ether, diisopropyl ether, dimethyl ether, benzene, toluene, cyclohexane, xylene or mixture thereof.

4. A process for purification of Chlorpromazine hydrochloride of formula Ia 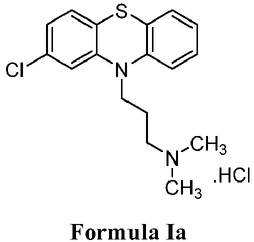 said process comprising the steps of:
(i) providing a solution of Chlorpromazine hydrochloride of formula Ia in one or more organic solvent;
(ii) optionally concentrating the solution obtained in step (i) under reduced pressure;
(iii) adding ester solvent to the solution or suspension obtained in step (i) or (ii);
(iv) optionally cooling the step (iii); and
(v) isolating the Chlorpromazine hydrochloride of formula Ia from the solution or suspension obtained in step (iii) or (iv).

5. The process as claimed in claim 4, wherein said organic solvent is selected from the group comprising of xylene, toluene, benzene, hexane, cyclohexane, methyl cyclohexane, dichloromethane, chloroform, carbon tetrachloride, dichloromethane, dichloroethane, dimethyl formamide, acetone, methanol, ethanol, isopropanol, sulfolane, dimethyl sulfoxide or mixture thereof; preferably mixture of toluene and methanol.

6. The process as claimed in claim 4, wherein said ester solvent is selected from the group comprising of ethyl acetate, butyl acetate, methyl acetate, isopropyl acetate, isoamyl acetate, ethyl butyrate, ethyl acetoacetate or mixture thereof; preferably ethyl acetate.

7. A process for the preparation of Chlorpromazine hydrochloride compound of formula Ia having less than 0.05% w/w of dimeric impurities of formula IVa or IVb, 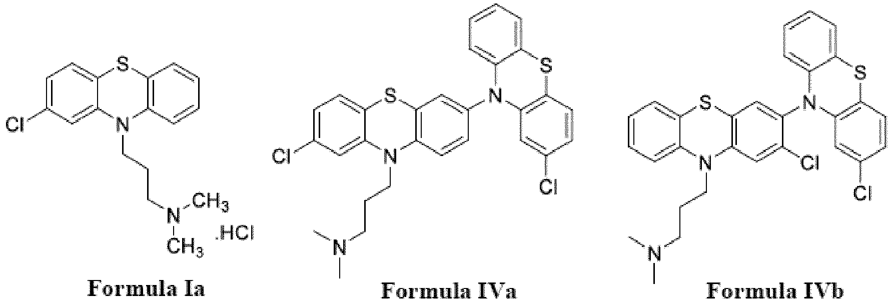 said process comprising the steps of:
(i) reacting 2-chlorophenothiazine compound of formula II with 3-dimethylaminopropylchloride compound of formula IIIa 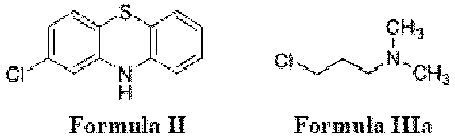 in the presence of base in biphasic medium to obtain Chlorpromazine;
(ii) providing a solution of Chlorpromazine in one or more organic solvent;
(iii) adding alcoholic hydrochloric acid to the solution obtained in step (ii);
(iv) isolating the Chlorpromazine hydrochloride of formula Ia from the solution obtained in step (iii); and
(v) optionally purifying the Chlorpromazine hydrochloride with one or more organic solvent.

8. The process as claimed in claim 7, wherein said Chlorpromazine hydrochloride is having the dimeric impurities of formula IVa or IVb less than 0.02% w/w.

9. The process as claimed in claim 7, wherein said base is selected from the group comprising of sodium hydroxide, potassium hydroxide, lithium hydroxide, magnesium hydroxide; carbonate or bicarbonates of sodium, potassium, magnesium and lithium or aqueous mixture thereof; preferably aqueous potassium hydroxide.

10. The process as claimed in claim 7, wherein said biphasic medium is selected from the group comprising of aqueous phase, non-aqueous phase selected from dichloromethane, dichloroethane, chloroform, carbon tetrachloride, tetrahydrofuran, diethyl ether, diisopropyl ether, dimethyl ether, benzene, toluene, cyclohexane, xylene or mixture thereof.

11. The process as claimed in claim 7, wherein said organic solvent in step (ii) is selected from the group comprising of xylene, toluene, benzene, hexane, cyclohexane, methyl cyclohexane, dichloromethane, chloroform, carbon tetrachloride, dichloromethane, dichloroethane, dimethyl formamide, acetone, methanol, ethanol, isopropanol, sulfolane, dimethyl sulfoxide or mixture thereof; preferably mixture of toluene and methanol.

12. The process as claimed in claim 7, wherein said alcoholic hydrochloric acid is selected from the group comprising hydrochloric acid of methanol, ethanol or isopropanol; preferably methanolic hydrochloric acid.

13. A process for preparation of compound of formula Ic 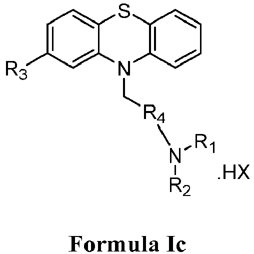 wherein R₁ and R₂ is selected from H or CH₃; R₃ is selected from H, Cl, Br, CF₃, CH₃ or OCH₃; R₄ is selected from substituted or unsubstituted C1-C4 alkyl such as methylene, ethylene, propylene or butylene; and X is selected from H, Cl, Br or I
said process comprising the step of:
a) reacting a compound of formula IIa wherein R₃ is selected from H, Cl, Br, CF₃, CH₃ or OCH₃ 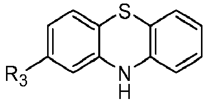 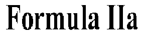 with a compound of formula IIIb wherein R1 and R2 is selected from H or CH₃; R4 is selected from substituted or unsubstituted C₁-C₄ alkyl such as methylene, ethylene, propylene, and butylene; and X is selected from H, Cl, Br, and I 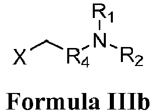 in the presence of base in a biphasic medium to obtain a compound of formula Ib; and 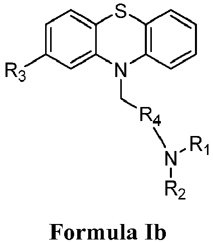
b) converting the compound of formula 1b to the compound of formula Ic.

14. The process as claimed in claim 13, wherein the compound of formula Ic is selected from Promethazine, Alimemazine, Acepromazine, Promazine, Chlorpromazine or the like

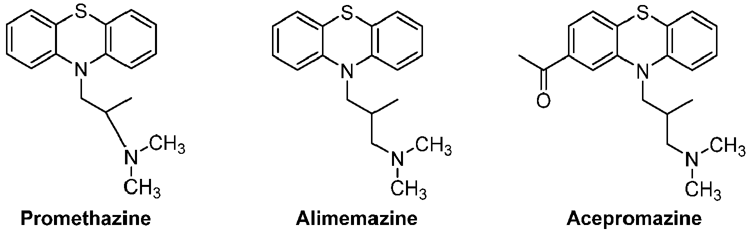

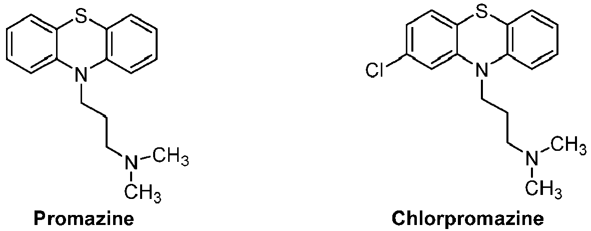

15. The process as claimed in claim 13, wherein said base is selected from the group comprising of sodium hydroxide, potassium hydroxide, lithium hydroxide, magnesium hydroxide; carbonate or bicarbonates of sodium, potassium, magnesium and lithium or aqueous mixture thereof; preferably aqueous potassium hydroxide.

16. The process as claimed in claim 13, wherein said biphasic medium is selected from the group comprising of aqueous phase, non-aqueous phase selected from dichloromethane, dichloroethane, chloroform, carbon tetrachloride, tetrahydrofuran, diethyl ether, diisopropyl ether, dimethyl ether, benzene, toluene, cyclohexane, xylene or mixture thereof.
